# EUROPEAN PATENT SPECIFICATION

(11) **EP 2 906 218 B1**
(45) Date of publication and mention of the grant of the patent: **14.12.2016**
(21) Application number: 13773749.0
(22) Date of filing: 07.10.2013
(51) Int. Cl.: A61K 31/4745, A61K 31/538, A61K 45/06, A61P 11/14

(54) **BETA-2-ADRENOCEPTOR AGONIST FOR THE TREATMENT OF COUGH**
BETA-2-ADRENOZEPTORAGONIST ZUR BEHANDLUNG VON HUSTEN
AGONISTE DES RÉCEPTEURS 2-ADRÉNERGIQUES UTILISÉ DANS LE TRAITEMENT DE LA TOUX

(30) Priority: 09.10.2012 EP 12187761
(43) Date of publication of application: 19.08.2015
(73) Proprietor: Boehringer Ingelheim International GmbH, 55216 Ingelheim am Rhein (DE)
(72) Inventor: BOUYSSOU, Thierry, 55216 Ingelheim am Rhein (DE); PIEPER, Michael Paul, 55216 Ingelheim am Rhein (DE)
(74) Representative: Simon, Elke Anna Maria
(86) International application number: PCT/EP2013/070812
(87) International publication number: WO 2014/056840

(56) References cited:
- EP-A1- 2 422 786
- US-A1- 2007 088 030
- PETER V DICPINIGAITIS ET AL: "Effect of Tiotropium on Cough Reflex Sensitivity in Acute Viral Cough", LUNG, SPRINGER-VERLAG, US, vol. 186, no. 6, 12 September 2008 (2008-09-12), pages 369-374, XP019657458, ISSN: 1432-1750, DOI: 10.1007/S00408-008-9114-6
- MARIO MALERBA ET AL: "Therapeutic potential for novel ultra long-acting [beta]2-agonists in the management of COPD: biological and pharmacological aspects", DRUG DISCOVERY TODAY, vol. 17, no. 9-10, 1 May 2012 (2012-05-01) , pages 496-504, XP55042135, ISSN: 1359-6446, DOI: 10.1016/j.drudis.2011.11.002

## Description

This invention relates to the ß2-adrenoceptor agonist Olodaterol or a pharmaceutically acceptable salt thereof, 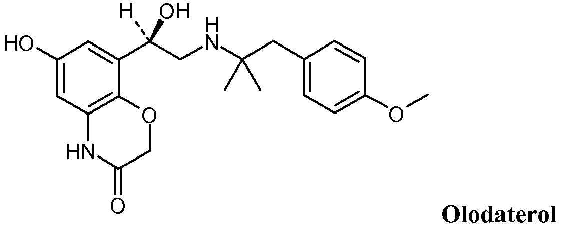 for use in a method of treatment of cough from various origins.

### BACKGROUND INFORMATION

Cough is an important defensive reflex of the airway and also a common symptom of respiratory disease. Cough after common respiratory virus infection is transient but is more persistent when associated with conditions such as asthma, chronic obstructive pulmonary disease COPD), idiopathic pulmonary fibrosis (IPF) and cancer.

Although cough is a protective mechanism, chronic and uncontrollable cough belongs to the most debilitating symptoms found in patients with chronic airway diseases (Lee and Undem, 2004). Chronic cough is experienced by more than 16 % of the population and represents one of the most complaints that general practitioners or respiratory specialists are confronted with.

The most common anti-tussive treatments are represented by opioids such as codeine, dextromethorphan and noscapine. But, these drugs produce side-effects such as drowsiness, nausea, constipation and physical dependence, at their therapeutic doses.

In conditions such as asthma, COPD or IPF, inhaled therapy of once-daily ß2-adrenoceptor agonist like Indacaterol, enhances the cough reflex (Pharmacother. 2012;32(5):456-74) while shorter acting ß2-adrenoceptor agonists like Formoterol and Salmeterol display anti-tussive effect at their therapeutic doses (Drug Research. 2008;58(4):168-73).

Olodaterol is a once-daily ß2-adrenoceptor agonist known from EP1562603. Preparation and formulations of Olodaterol or combinations of Olodaterol with Tiotropium salts, especially Tiotropium bromide are known from the prior art, exemplary cited are EP1809293, EP1809236, EP1940349, EP1917253 and EP2125759.

### BRIEF SUMMARY OF THE INVENTION

It was surprisingly found, that the once daily long acting ß2-adrenoceptor agonist Olodaterol has the particularity to exert anti-tussive activity at its full effective dose, but also at its minimum effective dose for bronchoprotection (0.01 µg/kg i.t.).

Furthermore Olodaterol causes no addiction or arrhythmia, especially not at the low doses where it is showing the anti-tussive effect.

In a further aspect, Olodaterol does not increase heart rate at doses including its full effective dose for bronchoprotection and at doses showing the anti-tussive effect.

### DETAILED DESCRIPTION OF THE INVENTION

The invention is related to Olodaterol or a pharmaceutically acceptable salt thereof, for use in a method of treatment of cough.

Preferably for use in a method of treatment of cough concomitant to viral infection, asthma, allergen-induced asthmatic reactions, cystic fibrosis, bronchitis, chronic bronchitis, chronic obstructive pulmonary disease (COPD), adult respiratory distress syndrome (ARDS), chronic pulmonary inflammation, rhinitis, allergic rhinitis, upper respiratory tract inflammatory disorders (URID), ventilator induced lung injury, silicosis, talcosis, pulmonary sarcoidosis, idiopathic pulmonary fibrosis (IPF) or bronchopulmonary dysplasia.

Specific types of coughs which may be treated by compounds of the present invention, include, but are not limited to dry cough, wet cough, croupy cough, or chest cough. Preferred for the above mentioned use is a pharmaceutically active salt of Olodaterol, especially Olodaterol hydrochloride.

Accordingly, in a further aspect, the present invention provides the use of olodaterol in a method for treating cough in a patient comprising administering olodaterol to said patient. In a further aspect, the administration of olodaterol does not cause addiction or arrhythmia in said patient. In a further aspect, the heart rate of the patient is not increased.

In a further aspect, the present invention provides treating a disease treatable by a bronchoprotective medicament comprising identifying a patient in need of said medicament and at risk of arrhythmia and administering olodaterol to said patient.

In a further aspect, the present invention provides a method of treating a disease treatable by a bronchoprotective medicament comprising determining that a patient in need of said medicament is also at risk of arrhythmia and administering olodaterol to said patient.

In a further aspect, a full effective dose of olodaterol is administered to said patient. In a further aspect olodaterol is administered to said patient based on the recognition of the safety margin of olodaterol. In a further aspect, olodaterol is administered to said patient based on the ratio dose inducing side-effect/ bronchoprotective FED of olodaterol.

### COMBINATIONS

Olodaterol is known as combination partner of Tiotropium salts, especially Tiotropium bromide, from EP 1781298. Additionally Tiotropium bromide is also available for the use as a medicament for the treatment of cough (see Lung. 2008, 186:369-74).

Thus, another aspect of the invention is Olodaterol or a pharmaceutical acceptably salt thereof in combination with Tiotropium or a pharmaceutical acceptable salt thereof, preferably tiotropium bromide, for use as a medicament for the treatment of cough. Whereas the application of the combination can occur as a fixed dose combination or as free dose combination simultaneously or sequentially.

In the preferred use for the treatment of cough according to the invention it is particularly preferred to use preparations or pharmaceutical formulations which are suitable for inhalation. Inhalable preparations include inhalable powders, propellant containing metered-dose aerosols or propellant free inhalable solutions. Within the scope of the present invention, the term propellant free inhalable solutions also include concentrates or sterile ready-to-use inhalable solutions. The formulations which may be used within the scope of the present invention are known from the above mentioned prior art.

### DOSAGE

The dose range of Olodaterol applicable per day is usually from 0.01 to 50 µg, preferably from 0.05 to 25 µg, more preferably from 1 to 10 µg, most preferably 1, 2.5, 5 or 10 µg. Olodaterol fully effective dose in human in watery solution by inhalation with Respimat (see EP1809293) is comprised between 2.5 and 10 µg/day.

A once daily application of the full effective dosage of Olodaterol or a salt thereof is preferred. Accordingly, in one aspect of the present invention, 5 µg of Olodaterol is administered daily to the patient, for example through 2 actuations from the mouthpiece of a delivery device, each actuation containing 2.7 µg olodaterol hydrochloride, equivalent to 2.5 µg olodaterol. Nevertheless a dosage unit may also contain half of the fully effective dose, then a once daily treatment by sequenced application of this half dose unit is preferred. Accordingly, in another aspect of the present invention, only one actuation from the mouthpiece containing 2.7 µg olodaterol hydrochloride, equivalent to 2.5 µg olodaterol, is administered to a patient daily. Alternatively, in a further aspect of the present invention two actuations from the mouthpiece each containing 1.35 µg olodaterol hydrochloride, equivalent to 1.25 µg olodaterol, are administered to a patient daily.

In combination with Tiotropium salts the dose range of Olodaterol is the same than above and the dose range of the Tiotropium salt, e.g. Tiotropium bromide applicable per day is usually from 1 to 50 µg, preferably from 1 to 30 µg, more preferably from 1 to 20 µg, most preferably 1, 2.5, 5, 10 or 18 µg. Tiotropium fully effective dose in combination by inhalation of a powder formulation (e.g. Handyhaler from EP 1940349) is 18 µg/day and in watery solution by inhalation with Respimat (see EP1940349) is between 2.5 and 5 µg/day. Olodaterol fully effective dose is the same than above.

A once daily application of the full effective dosage of Olodaterol and Tiotropium or salts thereof according to the above description is preferred. Nevertheless a dosage unit may also contain half of the fully effective dose, then a once daily treatment by sequenced application of this half dose unit is preferred.

The actual pharmaceutically effective amount or therapeutic dosage will of course depend on factors known by those skilled in the art such as age and weight of the patient, route of administration and severity of disease. In any case the combination will be administered at dosages and in a manner which allows a pharmaceutically effective amount to be delivered based upon patient's unique condition.

### EXAMPLES

Other features and advantages of the present invention will become apparent from the following more detailed examples which illustrate, by way of example, the principles of the invention.

### Anti-tussive activity

*Animals -* Male albino Dunkin-Hartley guinea pigs (350 - 550g; Charles River WIGA GmbH, Sulzfeld, Germany) were used. The animals were housed in groups of 6 in solid floor cages and allowed free access to water and standard food. The guinea pigs were kept in rooms maintained at a constant temperature (22°C ± 2°C) and humidity (60% ± 15%), with a 12h light cycle. The experiments were approved by the ethical committee Regierungspräsidium Tübingen, Germany.

*OVA asthma model -* On days 1 and 2, the guinea pigs were immunised by subcutaneous injection of 0.5ml per animal of a solution of ovalbumin (OVA) (40 µg/ml) and aluminium hydroxide [A1 (OH)₃]. On day 14, these sensitised guinea pigs were challenged with OVA by exposure to an OVA aerosol (1.25 mg/ml) for 5 minutes, using a Dräger Inhalette® (Dräger Medical AG & Co. KgaA, Lübeck, Germany). 30 minutes prior to the challenge, pyrilamine maleate (2 mg/kg i.p.) was administered to protect the animals against bronchospasm. On day 15, the citric acid challenge was given to induce cough. 2 hours before the challenge, the test compound or vehicle (saline) was delivered by intratracheal (i.t.) administration (0.5 ml/kg) under anaesthesia with isoflurane (Forene®, Abbott GmbH and CO. KG, Wiesbaden, Germany). Tested compounds were:
- Codeine: 1, 3, 10, and 30 mg/kg i.t. (codeine was used as gold standard for the validation of the experiment)
- Olodaterol: 0.01, 0.03, 0.1, 0.3, 1, and 3 µg/kg i.t.
- Formoterol: 1,3, and 10 µg/kg i.t.
- Indacaterol: 3, 10, and 30 µg/kg i.t.

*Cough recording-* Two hours after i.t. application, the citric acid challenge was performed. A whole body plethysmograph manufactured for guinea pigs (Buxco Research Systems, Wilmington, NC, USA) was used for the experiments. Conscious animals were individually placed, unrestrained, into a whole body plethysmograph chamber. A solution of citric acid (0.4 M) was nebulised using the Aerosol Delivery System (Buxco) and administered to the animals at a rate of nebulisation of 0.18ml/min for 15 min. During the challenge, airflow in the chamber was recorded by the Buxco system and analysed for the transient increase in airflow produced by cough, in which rapid inspiration is followed by rapid expiration. The system was able to distinguish coughs from other expiratory responses, and the number of coughs was counted.

*Data analysis -* Data from the treatment groups were compared with the vehicle group. Data are expressed as mean ± SEM of coughs produced by guinea pigs within each group during a 15 minutes citric acid aerosol exposure. Mean values were statistically analyzed by one-way analysis of variance (ANOVA) followed by the Dunnett's Multiple comparisons Test to evaluate significant differences between groups with p < 0.05 being considered significant.

### Results

### Olodaterol

| treatment | Number of coughs | SEM | Inhibition of cough (%) vs vehicle | Number of animals |
|---|---|---|---|---|
| Vehicle | 24 | 2 | - | 12 |
| 0.01 µg/kg | 16 | 2 | 33 | 6 |
| 0.03 µg/kg | 10 | 6 | 58 * | 6 |
| 0.1 µg/kg | 5 | 4 | 79 * | 6 |
| 0.3 µg/kg | 5 | 4 | 79 * | 6 |
| 1 µg/kg | 6 | 6 | 75 * | 6 |
| 3 µg/kg | 5 | 3 | 79 * | 6 |

| | | | | |
|---|---|---|---|---|
| *p < 0.05 olodaterol versus vehicle | | | | |

### Codeine

| treatment | Number of coughs | SEM | Inhibition of cough (%) vs vehicle | Number of animals |
|---|---|---|---|---|
| Vehicle | 24 | 2 | - | 12 |
| 1 mg/kg | 20 | 10 | 17 | 6 |
| 3 mg/kg | 14 | 2 | 42 * | 6 |
| 10 mg/kg | 10 | 4 | 58 * | 6 |
| 30 mg/kg | 7 | 3 | 71 * | 6 |

| | | | | |
|---|---|---|---|---|
| *p < 0.05 codeine versus vehicle | | | | |

### Formoterol

| treatment | Number of coughs | SEM | Inhibition of cough (%) vs vehicle | Number of animals |
|---|---|---|---|---|
| Vehicle | 24 | 2 | - | 12 |
| 1 µg/kg | 26 | 3 | 0 | 6 |
| 3 µg/kg | 7 | 3 | 71 * | 6 |
| 10 µg/kg | 10 | 4 | 58 * | 6 |

| | | | | |
|---|---|---|---|---|
| *p < 0.05 formoterol versus vehicle | | | | |

### Indacaterol

| treatment | Number of coughs | SEM | Inhibition of cough (%) vs vehicle | Number of animals |
|---|---|---|---|---|
| Vehicle | 24 | 2 | - | 12 |
| 3 µg/kg | 26 | 6 | 0 | 6 |
| 10 µg/kg | 32 | 10 | 0 | 6 |
| 30 µg/kg | 38 | 11 | 0 | 6 |

### Bronchoprotection

*Animal -* Male and female Dunkin-Harley guinea pigs were obtained from the Experimental Animal Breeding Centre of Harlan Winkelmann (Germany). After fasting overnight, but with free access to drinking water, animals with body weight of 400 - 500 g are used.

*Anesthesia and preparation -* The anesthesia was induced by intraperitoneal injection of 50 mg/kg pentobarbital. Anesthesia was prolonged by intravenous infusion of pentobarbital (15 mg/kg/h) via the jugular vein. A tracheal cannula was introduced after tracheotomy for artificial ventilation. The internal jugular vein was cannulated for acetylcholine injection.

*Instrumentation -* Bronchospasm was recorded with a modified version of the method of Konzett-Rößler described by Walland et al, 1997. The animals were ventilated by means of a piston pump (starling ventilator, Hugo Sachs Elektronik, Germany) at a stroke volume of 1 ml/100 g body weight and at a rate of 60 strokes per min. The tubing which connected the tracheal cannula with the ventilator was provided with a branch leading to the bronchospasm transducer (bronchospasm transducer 7020, Ugo Basile, Italy). Lung resistance was measured as ml of overflow and was recorded, amplified and saved under Notocord files (Notocord-hem, Notocord, France). Blood pressure and heart rate were monitored from a carotid artery in order to check the anesthesia and the variability of the preparation.

*Experimental protocol -* 2 hours before acetylcholine challenge, guinea pigs were given the test compound or its vehicle intra-tracheally under a slight isoflurane anesthesia. 30 minutes before acetylcholine challenge, lung resistance and blood pressure were measured under anesthesia. At t: 0, acetylcholine was injected intravenously at a fixed dose of 14 µg/kg. Compounds were tested at doses of:
- Olodaterol: 0.01, 0.03, 0.1, 0.3, and 1 µg/kg
- Formoterol: 0.1, 0.3, 1, and 3 µg/kg
- Indacaterol: 3, 10, and 30 µg/kg
At the end of the experiment, animals were euthanized by an overdose of pentobarbital (100 mg/kg i.v.).

*Data analysis -* Data from the treatment groups were compared with the vehicle group. Data are expressed as mean ± SD of ml of overflow. Bronchoprotection was expressed as percentage of inhibition of the bronchoconstriction recorded with acetycholine at 14 µg/kg i.v. Mean values were statistically analyzed by one-way analysis of variance (ANOVA) followed by the Dunnett's Multiple comparisons Test to evaluate significant differences between groups with p < 0.05 being considered significant.

### Results

### Olodaterol

| Treatment | Bronchoconstriction (ml overflow) | SD | Bronchoprotection (%) | Number of animals |
|---|---|---|---|---|
| Vehicle | 37 | 11 | - | 12 |
| 0.01 µg/kg | 25 | 12 | 32 | 6 |
| 0.03 µg/kg | 14 | 9 | 62 * | 6 |
| 0.1 µg/kg | 8 | 3 | 78 * | 6 |
| 0.3 µg/kg | 4 | 2 | 89 * | 6 |
| 1 µg/kg | 2 | 1 | 95 * | 6 |

| | | | | |
|---|---|---|---|---|
| *p < 0.05 olodaterol versus vehicle | | | | |

### Formoterol

| Treatment | Bronchoconstriction (ml overflow) | SD | Bronchoprotection (%) | Number of animals |
|---|---|---|---|---|
| Vehicle | 37 | 11 | - | 12 |
| 0.1 µg/kg | 26 | 12 | 29 | 6 |
| 0.3 µg/kg | 14 | 9 | 62 * | 6 |
| 1 µg/kg | 9 | 2 | 78 * | 6 |
| 3 µg/kg | 4 | 2 | 89 * | 6 |

| | | | | |
|---|---|---|---|---|
| *p < 0.05 formoterol versus vehicle | | | | |

### Indacaterol

| Treatment | Bronchoconstriction (ml overflow) | SD | Bronchoprotection (%) | Number of animals |
|---|---|---|---|---|
| Vehicle | 37 | 11 | - | 12 |
| 1 µg/kg | 34 | 9 | 8 | 6 |
| 3 µg/kg | 18 | 4 | 51* | 6 |
| 10 µg/kg | 6 | 3 | 84 * | 6 |
| 30 µg/kg | 5 | 2 | 86 * | 6 |

| | | | | |
|---|---|---|---|---|
| *p < 0.05 indacaterol versus vehicle | | | | |

### Summary

### Olodaterol

| treatment | Anti-tussive effect (%) | Bronchoprotection (%) |
|---|---|---|
| 0.01 µg/kg | 33 | 32 |
| 0.03 µg/kg | 58 | 62 |
| 0.1 µg/kg | 79 | 78 |
| 0.3 µg/kg | 79 | 89 |
| 1 µg/kg | 75 | 95 |

| | | |
|---|---|---|
| ED₅₀ anti-tussive activity: 0.02 µg/kg i.t. ED₅₀ bronchoprotection: 0.02 µg/kg i.t. | | |

### Formoterol

| Treatment | Anti-tussive effect (%) | Bronchoprotection (%) |
|---|---|---|
| 0.1 µg/kg | - | 29 |
| 0.3 µg/kg | - | 62 |
| 1 µg/kg | - | 78 |
| 3 µg/kg | 71 | 89 |
| 10 µg/kg | 58 | - |

| | | |
|---|---|---|
| ED₅₀ anti-tussive activity: 2 µg/kg i.t. ED₅₀ bronchoprotection: 0.02 µg/kg i.t. | | |

### Indacaterol

| treatment | Anti-tussive effect (%) | Bronchoprotection (%) |
|---|---|---|
| 1 µg/kg | - | 8 |
| 3 µg/kg | 0 | 51 |
| 10 µg/kg | 0 | 84 |
| 30 µg/kg | 0 | 86 |

| | | |
|---|---|---|
| No anti-tussive effect up to 30 µg/kg i.t. ED₅₀ bronchoprotection: 3 µg/kg i.t. | | |

### Side-Effects and Comparison / Relation

In the following the heart rate is expressed as a percentage of the pre-value (heart beats per minute) preceding the drug administration. The increase in heart rate expressed in percent corresponds to the difference of heart rate between the vehicle group and the treated group recorded 20 minutes after drug instillation. An increase in heart rate of at least 10 % or more in the treated group compared to the vehicle group is considered to be relevant as a systemic pharmacological activity (arrhythmia), while an increase of less than 10% is not considered relevant.
- Dose inducing **side-effect** (increase in heart rate) is the first dose increasing heart rate of at least 10 % or more compared to the vehicle group recorded 20 min after administration of the drug: olodaterol: 30 µg/kg (+13 %); formoterol: 10 µg/kg (+21 %); indacaterol: 100 µg/kg (+15 %).
- **Anti-tussive:** Cough reflex is a defensive reflex aiming to expel foreign particles, therefore 20 % of the cough reflex should be maintained. FED is the first dose without side-effect and displaying 70 to 80 % efficacy: olodaterol: 0.1 µg/kg (+79 %); formoterol: 3 µg/kg (+71 %); indacaterol has no anti-tussive activity.
- **Bronchoprotection:** Bronchoconstriction is a deleterious pulmonary reaction that should be abolished completely. FED is the first dose without side-effect and displaying 80 to 100 % efficacy: olodaterol: 0.3 µg/kg (+89 %); formoterol: 3 µg/kg (89 %); indacaterol: 10 µg/kg (84 %).

**Olodaterol:** The first side-effects (increased heart rate) were measured at a dosage of 30 µg/kg (13 % increase from baseline, dose inducing side-effect).
- antitussive Fully Effective Dose (FED): 0.1 µg/kg
- bronchoprotective FED (see above): 0.3 µg/kg
- ratio dose inducing side-effect/antitussive FED: 300
- ratio dose inducing side-effect/bronchoprotective FED: 100

**Formoterol:** The first side-effects (increased heart rate) were measured at a dosage of 10 µg/kg (21 % increase from baseline, dose inducing side-effect).
- antitussive FED: 3 µg/kg
- bronchoprotective FED: 3 µg/kg
- ratio dose inducing side-effect/antitussive FED: 3.3
- ratio dose inducing side-effect/bronchoprotective FED: 3.3

**Indacaterol:** The first side-effects (increased heart rate) were measured at a dosage of 100 µg/kg (15 % increase from baseline, dose inducing side-effect).
- no antitussive effect
- bronchoprotective FED: 10 µg/kg
- ratio dose inducing side-effect/antitussive effect: -
- ratio dose inducing side-effect/bronchoprotective FED: 10

Thus, Olodaterol does not increase the heart rate (less than 10% increase) at doses including its full effective dose for bronchoprotection and at doses showing the anti-tussive effect. An increase in heart rate was observed at a dose 300 times higher than the antitussive Fully Effective Dose (FED) and 100 times higher than the bronchoprotective FED (ratios dose inducing side-effect/antitussive FED and dose inducing side-effect/bronchoprotective FED of 300 and 100, respectively).

The high ratios shown above for Olodaterol indicate that there is a high safety margin against side effects at FED for Olodaterol, especially in comparison with Formoterol. For Indacaterol, which has no anti-tussive effect, the side effect risk in view of bronchoprotection seems to be better than Formoterol but, the safety margin is nevertheless 10 times less than for Olodaterol.

## Claims

1. Olodaterol or a pharmaceutically acceptable salt thereof, for use in a method of treatment of cough.

2. A pharmaceutically active salt of Olodaterol for the use according to claim 1.

3. Olodaterol hydrochloride for the use according to claim 2.

4. Olodaterol hydrochloride for the use according to claim 3 in a dosage from 0.01 to 50 µg.

5. Olodaterol hydrochloride for the use according to claim 4 in a once daily, inhalative dosage.

6. Olodaterol or a pharmaceutically active salt thereof for the use according to claim 1 to 5, wherein the method comprises co-administration of a tiotropium salt.

7. Olodaterol or a pharmaceutically active salt thereof, for the use according to claim 6, wherein the tiotropium salt is tiotropium bromide.

8. Olodaterol or a pharmaceutically active salt thereof, for the use according to claim 7, wherein the tiotropium bromide is administered in a dosage from 1 to 50 µg.

9. Olodaterol or a pharmaceutically active salt thereof, for the use according to claim 8, wherein the tiotropium bromide is administered in a once daily, inhalative dosage.

## Patentansprüche

1. Olodaterol oder ein pharmazeutisch akzeptables bzw. annehmbares Salz hiervon zur Verwendung in einem Verfahren zur Behandlung von Husten.

2. Pharmazeutisch wirksames Salz von Olodaterol zur Verwendung nach Anspruch 1.

3. Olodaterolhydrochlorid zur Verwendung nach Anspruch 2.

4. Olodaterolhydrochlorid zur Verwendung nach Anspruch 3 in einer Dosierung von 0,01 bis 50 µg.

5. Olodaterolhydrochlorid zur Verwendung nach Anspruch 4 in einer einzelnen täglichen Inhalationsdosis.

6. Olodaterol oder ein pharmazeutisch wirksames Salz hiervon zur Verwendung nach Anspruch 1 bis 5, wobei das Verfahren die Co-Verabreichung eines Tiotropiumsalzes umfasst.

7. Olodaterol oder ein pharmazeutisch wirksames Salz hiervon zur Verwendung nach Anspruch 6, wobei das Tiotropiumsalz Tiotropiumbromid darstellt.

8. Olodaterol oder ein pharmazeutisch wirksames Salz hiervon zur Verwendung nach Anspruch 7, wobei das Tiotropiumbromid in einer Dosierung von 1 bis 50 µg verabreicht wird.

9. Olodaterol oder ein pharmazeutisch wirksames Salz hiervon zur Verwendung nach Anspruch 8, wobei das Tiotropiumbromid in einer einzelnen täglichen Inhalationsdosis verabreicht wird.

## Revendications

1. Olodatérol ou un sel pharmaceutiquement acceptable de celui-ci, pour son utilisation dans une méthode de traitement de la toux.

2. Sel pharmaceutiquement actif de l'olodatérol pour l'utilisation selon la revendication 1.

3. Chlorhydrate d'olodatérol pour l'utilisation selon la revendication 2.

4. Chlorhydrate d'olodatérol pour l'utilisation selon la revendication 3, à une dose de 0,01 à 50 µg.

5. Chlorhydrate d'olodatérol pour l'utilisation selon la revendication 4, sous la forme d'une seule dose à inhaler quotidiennement.

6. Olodatérol ou un sel pharmaceutiquement actif de celui-ci pour l'utilisation selon l'une quelconque des revendications 1 à 5, dans laquelle
la méthode comprend la co-administration d'un sel de tiotropium.

7. Olodatérol ou un sel pharmaceutiquement actif de celui-ci, pour l'utilisation selon la revendication 6, dans laquelle le sel de tiotropium est le bromure de tiotropium.

8. Olodatérol ou un sel pharmaceutiquement actif de celui-ci, pour l'utilisation selon la revendication 7, dans laquelle le bromure de tiotropium est administré à une dose de 1 à 50 µg.

9. Olodatérol ou un sel pharmaceutiquement actif de celui-ci, pour l'utilisation selon la revendication 8, dans laquelle le bromure de tiotropium est administré sous la forme d'une seule dose à inhaler quotidiennement.
